# DEMANDE DE BREVET EUROPEEN

(11) **EP 2 495 001 A1**
(43) Date de publication de la demande: **05.09.2012**
(21) Numéro de dépôt: 11156889.5
(22) Date de dépôt: 03.03.2011
(51) Int. Cl.: A61M 5/158

(54) **Inserteur de canule**

(71) Demandeur: DEBIOTECH S.A., 1004 Lausanne (CH)
(72) Inventeur: Da Ros, Jérôme, 74200, Thonon les Bains (FR); Neftel, Frédéric, 1005, Lausanne (CH)
(74) Mandataire: Grosfillier, Philippe

(57) **Abrégé**

Inserteur de canule comprenant un boîtier (5) ayant une extrémité distale destinée à venir à proximité de la peau d'un patient et une extrémité proximale opposée, un piston (1), un déclencheur (4), des moyens de rappel (3) disposés entre le piston (1) et le déclencheur (4), des moyens de retenue (9) destinés à fixer au moins temporairement le piston (1) sur le déclencheur (4), des moyens de libération (8) destinés à libérer le piston (1) du déclencheur (4); l'inserteur comprenant en outre une aiguille (10) fixée à un support (2) ; le piston (1) étant monté dans le boîtier (5) de manière à ce qu'une fois libéré du déclencheur (4) il atteigne une première position puis une deuxième position; ledit support (2) étant en outre entraîné par le piston (1) selon une direction correspondant à l'orientation principale (12) du boîtier (5) et à rendre possible la rotation de l'aiguille autour de ladite orientation principale (12), lors de la transition entre ladite première et ladite deuxième position

## Description

### Domaine de l'invention

L'invention concerne l'insertion transdermique et la fixation d'une canule préalablement à l'administration et/ou à l'aspiration d'un liquide à travers la canule.

### Etat de la technique

Un inserteur de canule tel que décrit dans EP 1 743 667 A2 ou US 6,607,509 B2 comprend un corps sensiblement cylindrique à l'intérieur duquel se meut un piston. En se déplaçant vers l'extrémité distale de l'inserteur, le piston entraîne la canule ainsi qu'une aiguille - agissant comme mandrin ― qui est disposée à l'intérieur de la canule. Une fois la canule mise en place, l'aiguille est retirée.

L'insertion de la canule et le retrait de l'aiguille constituent deux actions importantes dans le fonctionnement d'un inserteur de canule.
Dans les inserteurs de l'état de la technique, ces deux actions sont réalisées indépendamment l'une de l'autre. L'insertion peut être manuelle alors que le retrait de l'aiguille est automatique ou vice-versa.
A ce jour il n'existe pas de système suffisamment économique permettant de combiner ces deux actions, en particulier lorsque l'on souhaite insérer la canule à une vitesse relativement importante, ce qui offrirait l'avantage de simplifier l'ensemble, de diminuer le temps d'intervention et d'augmenter la sécurité. En outre, les inserteurs de l'état de la technique ne permettent pas d'insérer une canule et de la fixer aisément sur un patch et/ou de réaliser une telle fonction à l'aide d'un dispositif suffisamment économique pour être conçu comme usage unique.

Il est donc hautement souhaitable d'avoir un inserteur de canule remédiant aux inconvénients précités.

### Description générale de l'invention

Un des buts de la présente invention est d'offrir la possibilité à l'utilisateur de réaliser la double opération « insertion de l'ensemble canule-aiguille » et « protection de l'aiguille » au moyen d'un même élément déclencheur, de préférence à usage unique.

Ce but est obtenu grâce à l'inserteur faisant l'objet des revendications ci-jointes.

L'inserteur de canule selon l'invention comprend un boîtier ayant une extrémité distale destinée à venir à proximité de la peau d'un patient et une extrémité proximale opposée, un piston , un déclencheur , des moyens de rappel disposés entre le piston et le déclencheur, des moyens de retenue destinés à fixer au moins temporairement le piston sur le déclencheur des moyens de libération destinés à libérer le piston du déclencheur; l'inserteur comprenant en outre une aiguille fixée à un support ; le piston étant monté dans le boîtier de manière à ce qu'une fois libéré du déclencheur il atteigne une première position puis une deuxième position; ledit support étant en outre entraîné par le piston selon une direction correspondant à l'orientation principale du boîtier et à rendre possible la rotation de l'aiguille autour de ladite orientation principale, lors de la transition entre ladite première et ladite deuxième position.

Les moyens de rappel peuvent être constitués d'un ressort ou d'un élastique.

Selon un mode de réalisation, l'extrémité distale des moyens de rappel fait office de piston.
Exprimé différemment, les moyens de rappel et le piston peuvent faire partie de la même pièce.

Les moyens de libération du piston peuvent être disposés en n'importe quel endroit permettant d'obtenir l'effet recherché. De préférence, il sont disposés sur la paroi interne du boîtier.

Le déclencheur peut se présenter de diverses manières, p.ex. sous forme de bouton activable par l'utilisateur.

De préférence, l'inserteur comprend un élément de sécurité qui empêche toute activation accidentelle du déclencheur.
L'élément de sécurité peut se présenter sous la forme de un ou deux boutons disposés sur la face latérale du boîtier. En appuyant sur le ou les boutons, le déclencheur n'est plus retenu de sorte que son activation est possible.

Alternativement, le déclencheur est libéré par déformation du boîtier.

La canule et son support peuvent être déplacés de différentes manières. Avantageusement, le piston comporte une face distale destinée à exercer exclusivement un effet de poussée sur la canule et son support. Dans cette variante, il n'existe donc pas de moyens de retenue (p.ex. des clips) qui fixeraient la canule et son support au piston.
Toujours dans le cadre de cette variante, la canule et son support sont fixés seulement à l'aiguille. Ce mode de fixation peut être obtenu par friction, entre l'aiguille et un septum disposé dans la canule.

L'insertion de la canule se fait par une action, à savoir une pression sur le déclencheur avec éventuellement, comme indiqué précédemment, le déclenchement préalable d'une sécurité qui retient le déclencheur. La rétraction (rotation) de l'aiguille usagée se fait de préférence automatiquement après son retrait du cathéter.
L'inserteur est retiré manuellement, une fois le retrait effectué, les moyens de rappel déplacent le piston vers sa deuxième position, ce qui a pour effet d'impartir une rotation à l'aiguille.

De préférence, le boîtier comporte un espace de protection dans lequel se loge l'aiguille rétractée.

Principalement, l'inserteur selon l'invention comprend les éléments suivants : un boîtier, un déclencheur de déclenchement, un piston, des moyens de rappel et une aiguille, cette dernière étant de préférence surmoulée dans un élément plastique, la partie proximale de cet élément constituant le support d'aiguille.
Le support d'aiguille est lié au piston de préférence par une liaison pivot.
Le déclencheur contient des éléments de rétention permettant de contenir un ressort comprimé entre le piston et le déclencheur.
La canule peut préférentiellement être orientée dans le boîtier et maintenue, p.ex. par l'aiguille.

L'inserteur est fixé au patch, par exemple au moyen d'un mécanisme à baïonnette. Dans cette configuration, l'inserteur peut avantageusement être utilisé comme poignée de préhension facilitant un positionnement adéquat du patch sur la peau.

Avant insertion de la canule, l'aiguille est guidée dans le boîtier pour empêcher sa rotation.
Après insertion de la canule, les éléments de butée du boîtier impartissent une rotation de l'aiguille et la contraignent à se loger dans l'espace du boîtier prévu à cet effet.

Avantageusement, l'inserteur selon l'invention comprend des éléments de sécurité qui empêchent une rotation inverse de l'aiguille une fois que celle-ci est logée dans l'espace du boîtier prévu à cet effet.

Toujours dans un contexte de sécurité, l'inserteur selon l'invention peut comporter des moyens qui empêchent l'activation du déclencheur tant que le patch n'est pas sur la peau. Ces moyens peuvent se présenter sous la forme d'un élément de forme allongée, monté coulissant à travers le patch et dont l'extrémité distale, lorsque le patch ne contacte pas la peau, émerge du patch du côté de la peau. Lors de la fixation du patch sur la peau, l'élément se rétracte dans le boîtier et son extrémité distale agit sur un mécanisme qui libère le déclencheur.

Une pression sur le déclencheur permet de libérer le piston, ainsi de libérer le ressort qui va faire translater le piston jusqu'à l'insertion de la canule à travers le patch et la peau. Dans cette première phase, l'aiguille agit comme mandrin qui facilite l'insertion de la canuler dans la peau.

Dans une deuxième phase, le retrait de l'inserteur entraîne également le retrait de l'aiguille de la canule, cette dernière restant insérée dans le patient. Pendant cette opération, le piston continue sa course en direction de l'extrémité distale de l'inserteur. Le piston se meut car il est entraîné par les moyens de rappel qui n'ont pas encore atteints leur position de repos. Ce mouvement du piston a pour effet simultané de libérer l'aiguille des guides du boîtier et la contraignent à effectuer une rotation.

### Description détaillée de l'invention

L'invention sera mieux comprise ci-après au moyen de quelques exemples illustrés. Il va de soi que l'invention ne se limite pas à ces modes de réalisation.

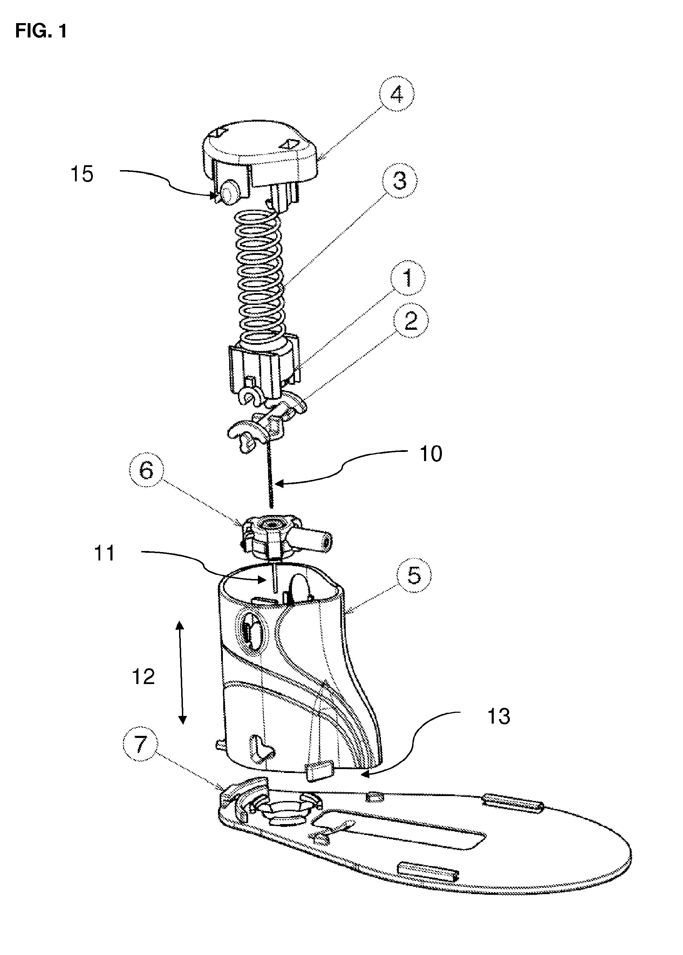

La figure 1 représente les différents composants de l'inserteur : Le piston **1**, l'aiguille surmoulée dans son support **2**, le ressort **3**, le déclencheur **4**, le boîtier **5**, la canule 11 et le patch **7**. Le déclencheur **4** comprend également au moins un bouton de sécurité **15**.
La figure 2 représente le piston **1** avec ses pinces de rétention **1a** permettant de clipser le support d'aiguille **2**, après assemblage des éléments, la rotation du support d'aiguille **2** est possible autour de l'axe **2a**.

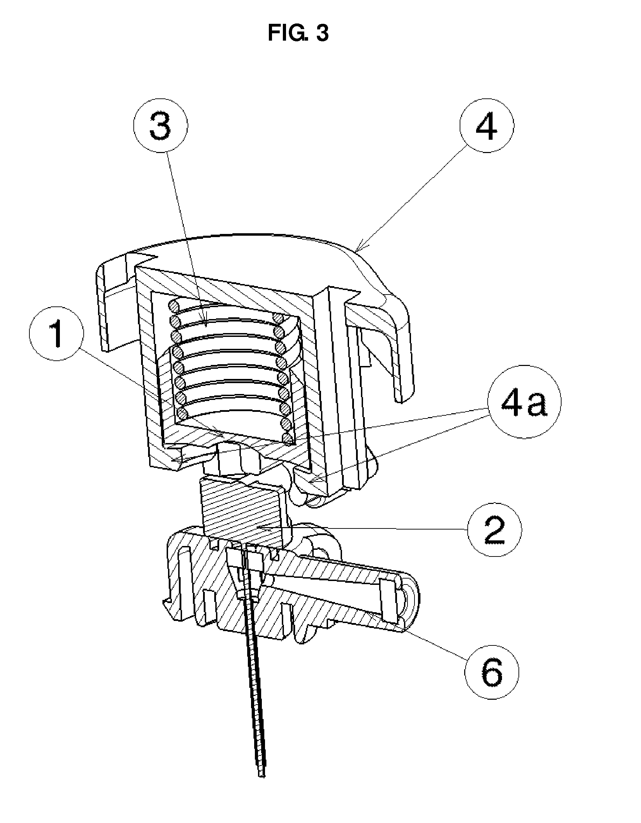

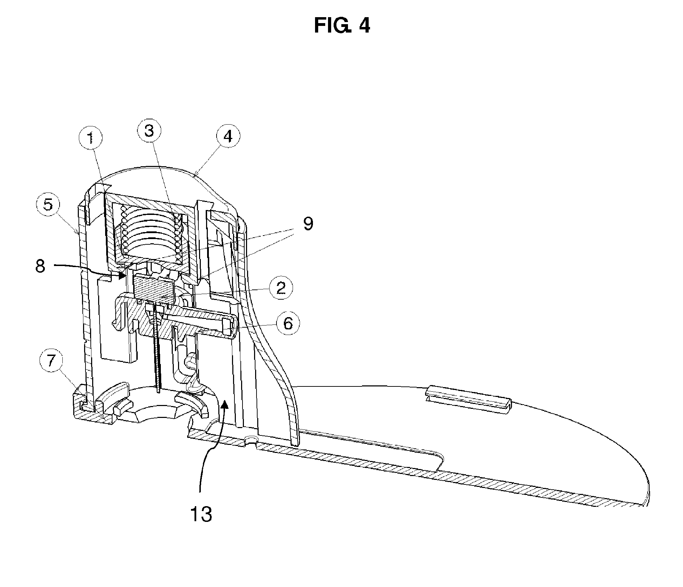

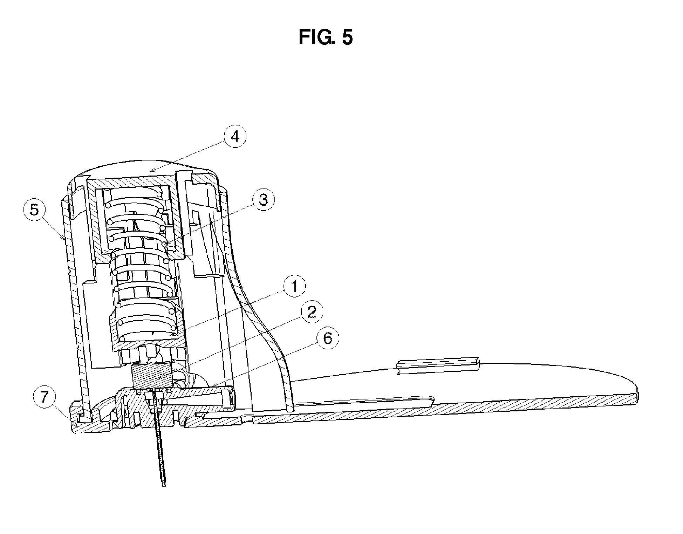

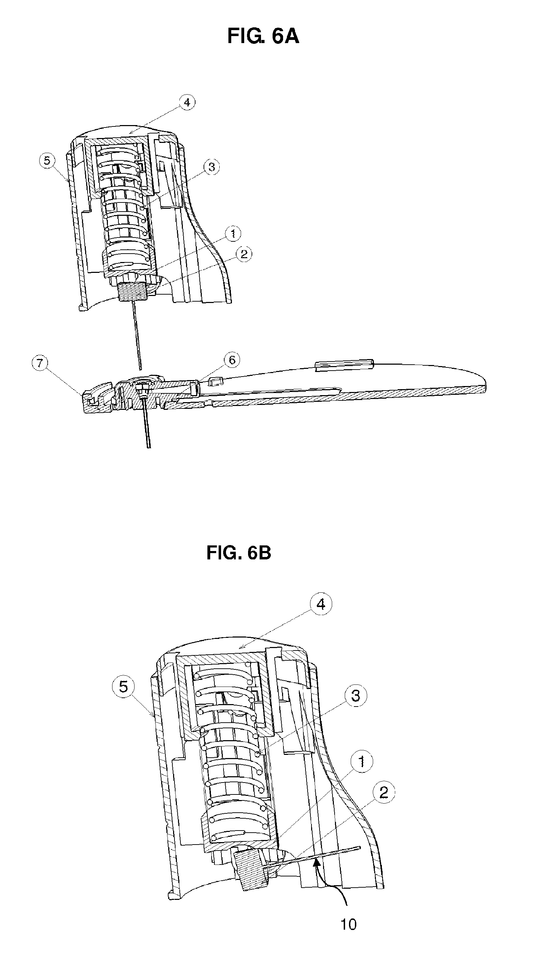

La figure 3 représente l'ensemble piston **1 -** déclencheur **4** avec le ressort comprimé **3** (tel que ce sous ensemble apparaît monté dans le boîtier **5** (cf. figure 4). Les pinces de rétention **9** permettent de maintenir le ressort **3** comprimé entre le piston inférieur **1** et le déclencheur **4**.
La figure 4 représente l'inserteur en position sur le patch 7 avant insertion, les pinces de rétention **9** permettent de garder le ressort **3** comprimé, de ce fait, l'inserteur est dans un état stable.

### Etapes d'insertion

### Figure 4

Une pression sur le déclencheur **4** a pour effet d'écarter les pinces de rétention **4a** et de libérer le piston **1**.

### Figure 5

Une première détente du ressort **3** entraîne l'insertion de l'aiguille **10** et de la canule **6** à travers le patch **7** et la peau du patient.

### Figure 6a, 6b

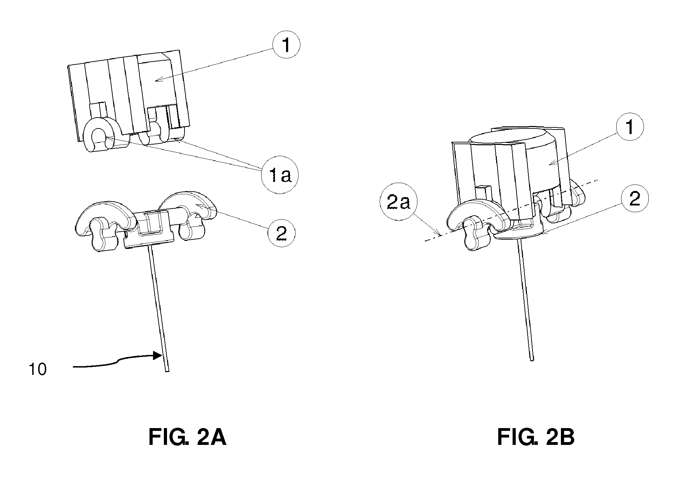

Dans un second temps, après le retrait de l'inserteur, le ressort **3** continue d'exercer une action sur le piston **1**. Dans son déplacement qui l'amène à sa position maximale, le piston **1** contraint l'aiguille et son support **2** à tourner autour de l'axe **2a** (figure2).

### Mécanisme d'insertion et de rotation de l'aiguille

### Figure 7a, 7b

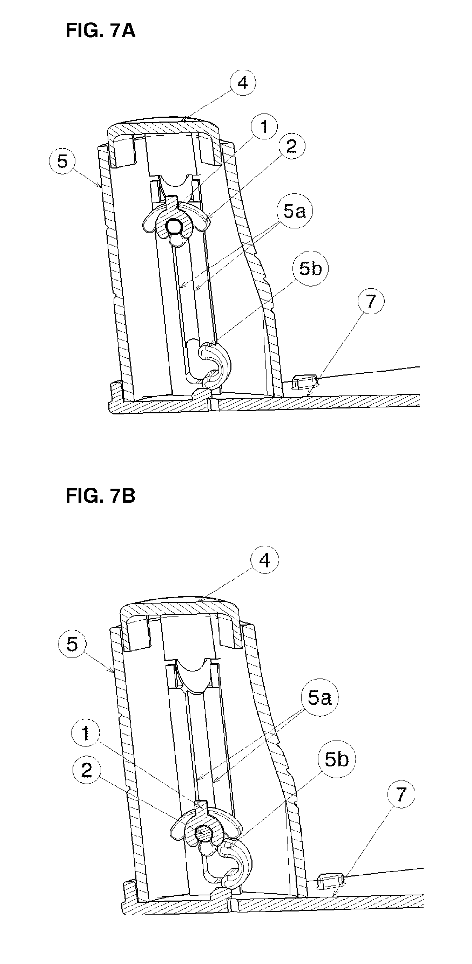

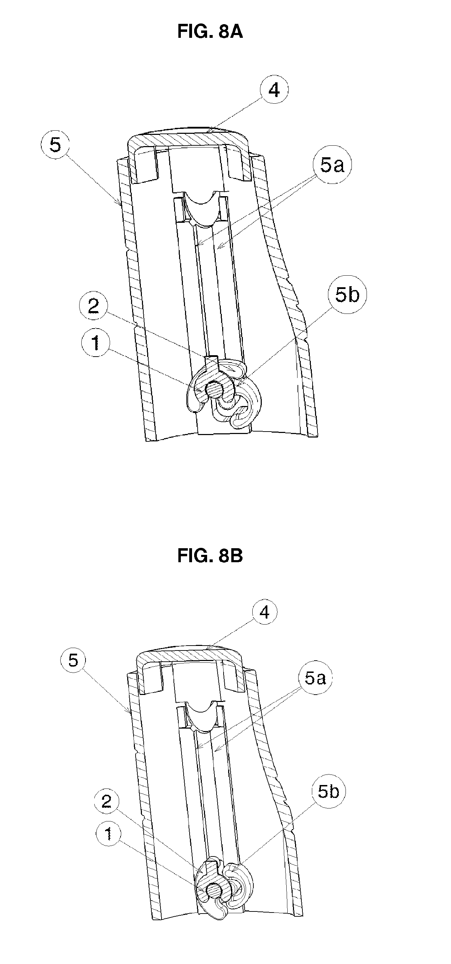

Pendant la phase d'insertion (représenté respectivement figure 7a à 7b), le support d'aiguille **2** est guidé dans le boîtier **5a** pour éviter sa rotation tout en étant maintenu par le piston **1**.

### Figure 8a, 8b

Après avoir manuellement retiré l'inserteur, l'action exercée par le ressort **3** sur le piston **1** et par conséquent sur le support d'aiguille **2** va contraindre le support d'aiguille contre le plot **5b** du boîtier, de ce fait, par design, obliger l'aiguille **2** à tourner, l'aiguille usagée est ainsi mise à l'écart à l'intérieur de l'inserteur.

### Figure 16

Cette figure représente le mécanisme de rotation de l'aiguille résultant du contact entre le support d'aiguille et des butées disposées dans le boîtier.

### Fixation de la canule sur le patch

La canule est conçue de sorte qu'elle soit p.ex. clipsée au patch lors de la phase d'insertion.

### Fixation momentanée du piston sur le déclencheur

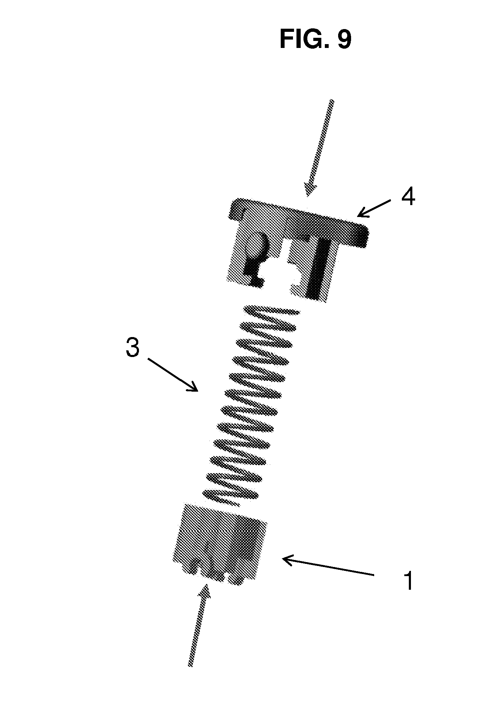

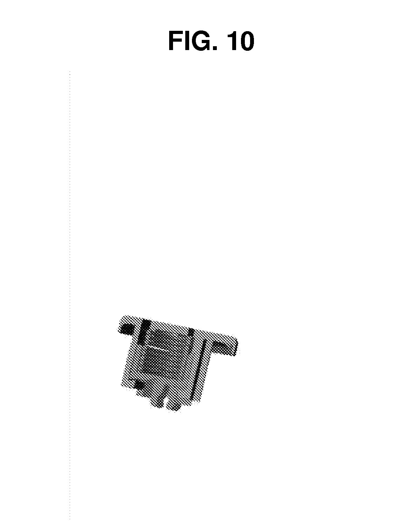

Cette étape est illustrée sur les figures 9 et 10. Une fois les pièces **4,1** fixées, le ressort **3** est comprimé entre celles-ci.

Cette étape lors de laquelle le ressort **3** est comprimé peut se produire avant le stockage de l'inserteur ou juste avant son utilisation.

### Fixation de la canule sur le piston

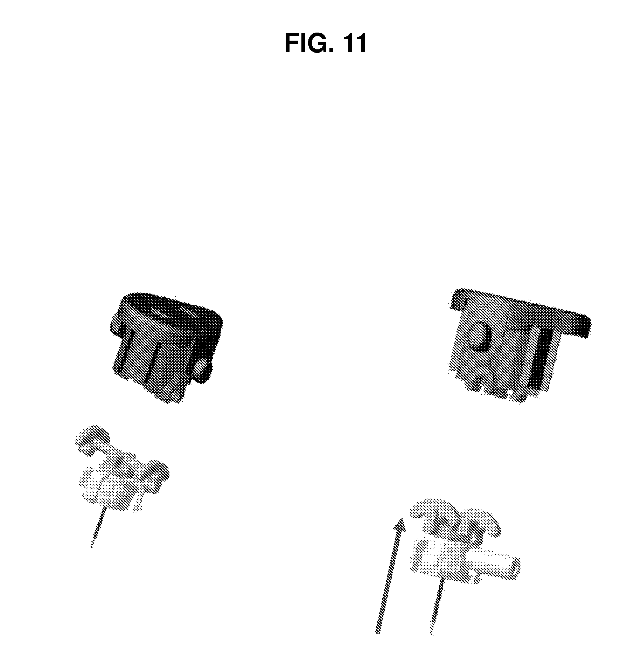

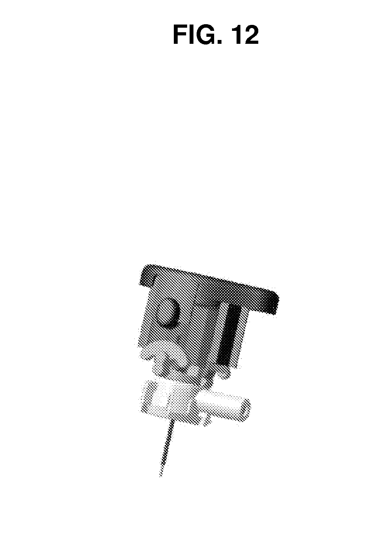

Cette étape est illustrée sur les figures 11 et 12.

### Insertion de l'ensemble déclencheur-piston-aiguille-canule dans le boîtier

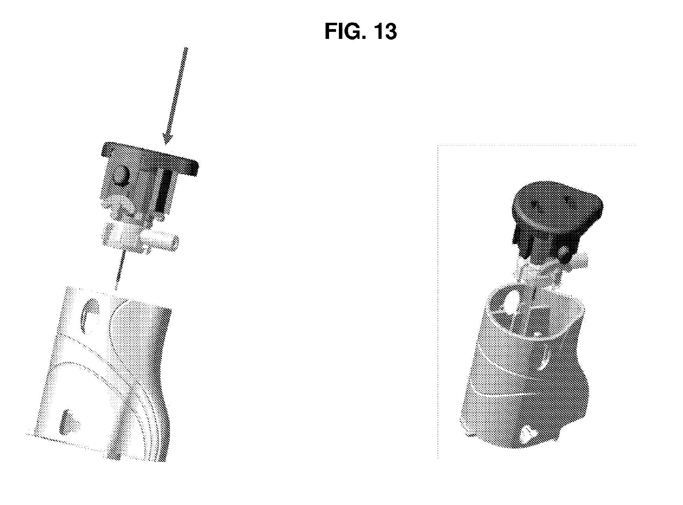

Cette insertion est illustrée sur la figure 13. A relever que l'ensemble est inséré du côté proximal de l'inserteur.
De préférence, l'aiguille et la canule sont disposés dans le boîtier **5** selon une direction parallèle à l'orientation principale **12** du boîtier **5**.
Il est cependant également possible de prévoir une configuration dans laquelle ces deux éléments sont disposés selon une direction qui forme un angle non nul par rapport à l'orientation principale **12** du boîtier **5**, afin d'insérer une aiguille dans la peau du patient avec un angle compris p.ex. entre 30 et 90°

### Fixation de l'inserteur sur le patch

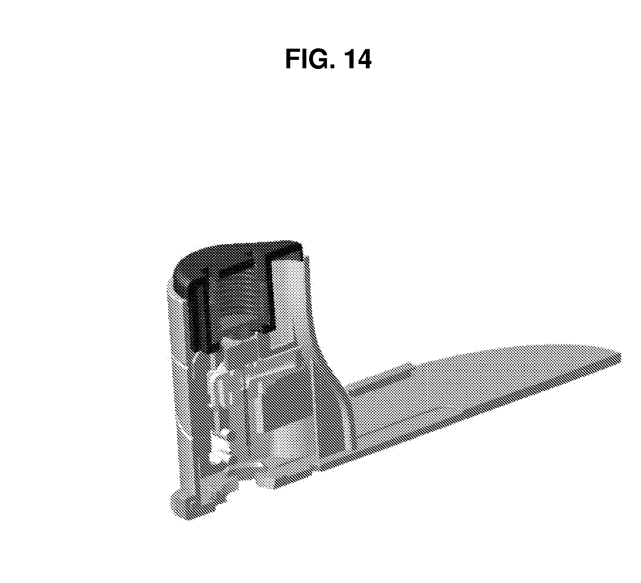

Cette dernière étape de préparation avant l'insertion de la canule est illustrée sur la figure 14. Dans cet exemple, l'inserteur est fixé sur le patch au moyen d'un mécanisme de baïonnette. Dans cette position, l'inserteur est prêt à l'emploi.

### Position de l'aiguille après usage

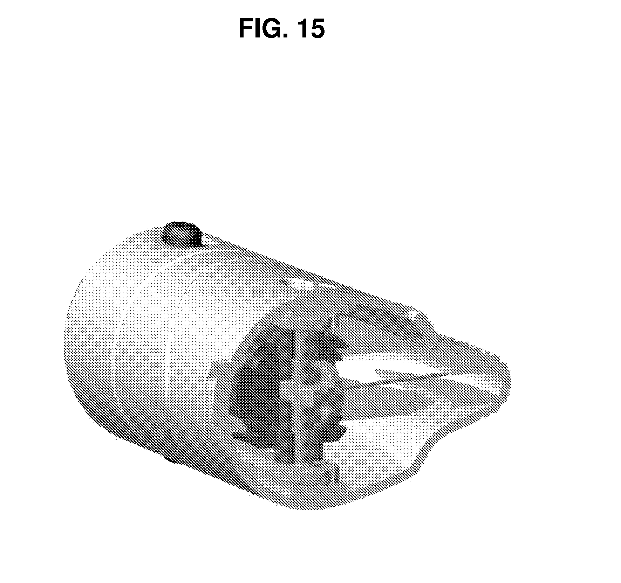

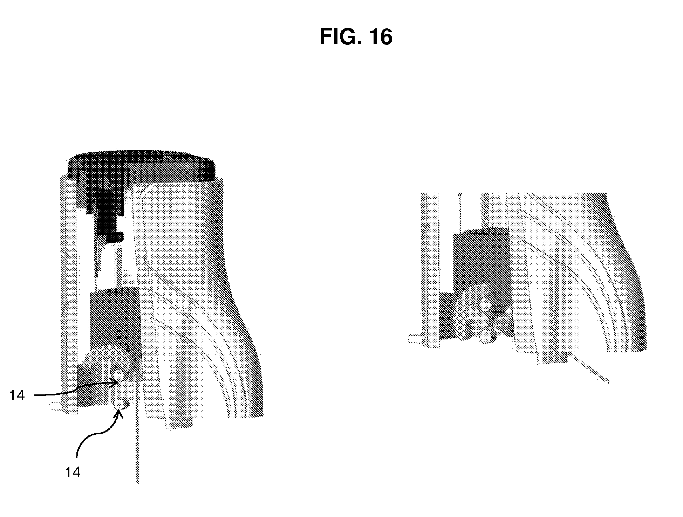

Cette étape finale est illustrée sur la figure 15.
Dans cette exemple, l'aiguille est dirigée selon une direction perpendiculaire par rapport à l'orientation principale du boîtier.

Il convient enfin de relever encore une fois que l'invention ne se limite pas aux exemples présentés dans cette demande.

Une telle canule peut notamment, mais non seulement, être utilisée pour l'injection d'insuline à un patient au moyen d'une pompe venant se fixer sur le patch qui rentre en connexion fluidique avec la canule.

### Liste des références numériques utilisées dans les figures :

- **1.**: Piston
- **1a.**: Pinces de rétention
- **2.**: Support d'aiguille
- **2a**: .Axe
- **3.**: Ressort
- **4.**: Déclencheur
- **5.**: Boîtier
- **5a.**: Guide
- **5b.**: Butée
- **6.**: Support de canule
- **7.**: **Patch**
- **8.**: Elément de libération
- **9.**: Pinces de rétention
- **10.**: Aiguille
- **11.**: Canule
- **12.**: Direction d'orientation principale du boîtier
- **14.**: xEspace de logement d'aiguille
- **14.**: Butée
- **15.**: Bouton de sécurité

## Revendications

1. Inserteur de canule comprenant un boîtier (5) ayant une extrémité distale destinée à venir à proximité de la peau d'un patient et une extrémité proximale opposée, un piston (1), un déclencheur (4), des moyens de rappel (3) disposés entre le piston (1) et le déclencheur (4), des moyens de retenue (9) destinés à fixer au moins temporairement le piston (1) sur le déclencheur (4), des moyens de libération (8) destinés à libérer le piston (1) du déclencheur (4); l'inserteur comprenant en outre une aiguille (10) fixée à un support (2) ; le piston (1) étant monté dans le boîtier (5) de manière à ce qu'une fois libéré du déclencheur (4) il atteigne et reste temporairement dans une première position, puis une deuxième position; l'aiguille (10) et son support (2) étant en outre disposés de manière à pouvoir être entraînés par le piston (1) selon une direction correspondant à l'orientation principale (12) du boîtier (5), avant que le piston (1) atteigne ladite première position, et de manière à pouvoir effectuer une rotation autour de ladite orientation principale (12) lors de la transition entre ladite première et ladite deuxième position.

2. Inserteur selon la revendication 1 dans lequel les moyens de rappel sont un ressort (3) ou un élastique.

3. Inserteur selon la revendication 1 ou 2 dans lequel les moyens de libération (8) sont disposés sur la paroi interne du boîtier (5).

4. Inserteur selon l'une des revendications précédentes dans lequel le déclencheur (4) est un bouton activable par un utilisateur.

5. Inserteur selon l'une des revendications précédentes dans lequel le boîtier (5) comprend des moyens de retenue du déclencheur (4), lesdits moyens étant adaptés pour libérer le déclencheur (4) consécutivement à une déformation du boîtier (5).

6. Inserteur selon l'une des revendications précédentes dans lequel le boîtier comporte un espace (13) destiné à loger intégralement ladite aiguille (10) lorsqu'elle est orientée selon une direction qui diffère de ladite orientation principale (12), le dit logement agissant comme protection de l'aiguille.

7. Inserteur selon l'une des revendications précédentes comprenant des éléments de butée (5b,14) disposés sur le boîtier (5) et sur le support d'aiguille (2), de manière à autoriser une rotation automatique de l'aiguille (3) dans ledit espace (13) lorsque le piston (1) se déplace entre ladite première position et ladite deuxième position.

8. Inserteur selon l'une des revendications précédentes comprenant un élément de sécurité (15) disposé de manière à retenir initialement le déclencheur (2).

9. Inserteur selon l'une des revendications précédentes dans lequel le piston (1) comporte une face distale destinée à exercer exclusivement un effet de poussée sur une canule (11) et son support (6).

10. Inserteur selon l'une des revendications 1 à 8 dans lequel la canule (11) et sont support (2) sont fixés au piston (1) pendant le déplacement vers l'extrémité distale du boîtier (5).

11. Inserteur selon la revendication 9 dans lequel la canule (11) et son support (2) sont fixés seulement à l'aiguille (10), de préférence par friction.

12. Inserteur selon la revendication 11 dans lequel un septum est disposé dans la canule, la fixation entre l'aiguille (10) et la canule (11) étant réalisé par friction entre l'aiguille (10) et le septum.

13. inserteur selon l'une des revendications précédentes dans lequel le boîtier (5) comporte des moyens de guidage (5a) pour guider l'aiguille (10) et/ou son support (2) lors de l'insertion de la canule (11).

14. Inserteur selon l'une des revendications précédentes dans lequel le boîtier (5) comporte des moyens de guidage pour guider le déplacement d'une canule (11) depuis l'extrémité proximale vers l'extrémité distale.

15. Ensemble comprenant un inserteur selon l'une des revendications 1 à 14 et une canule (11) comportant des clips ou autres moyens similaires disposés de manière à fixer la canule à un patch (7).

16. Ensemble selon l'une des revendications 14 dans lequel le boîtier (5) est configuré de manière à agir comme organe de préhension pour faciliter le positionnement d'un patch sur la peau du patient.

17. Ensemble comprenant un inserteur selon l'une des revendications 1 à 14 comprenant des moyens de sécurité qui empêchent l'activation du déclencheur (4) tant que le patch (7) n'est pas sur la peau.

18. Ensemble comprenant un inserteur selon l'une des revendications 1 à 14 comprenant un inserteur à usage unique.
